# EUROPEAN PATENT SPECIFICATION

(11) **EP 2 273 975 B1**
(45) Date of publication and mention of the grant of the patent: **09.04.2014**
(21) Application number: 09716304.2
(22) Date of filing: 02.03.2009
(51) Int. Cl.: A61K 9/08, A61K 31/4015

(54) **PHARMACEUTICAL SOLUTIONS, PROCESS OF PREPARATION AND THERAPEUTIC USES**
PHARMAZEUTISCHE LÖSUNGEN, HERSTELLUNGSVERFAHREN DAFÜR UND THERAPEUTISCHE ANWENDUNGEN DAVON
SOLUTIONS PHARMACEUTIQUES, PROCÉDÉ DE PRÉPARATION ET UTILISATIONS THÉRAPEUTIQUES

(30) Priority: 03.03.2008 EP 08003915
(43) Date of publication of application: 19.01.2011
(73) Proprietor: UCB Pharma, S.A., 1170 Brussels (BE)
(72) Inventor: SCHENKEL, Eric, 1070 Brussels (BE); POULAIN, Claire, 1070 Brussels (BE); DODELET, Bertrand, 1070 Brussels (BE); FANARA, Domenico, 1070 Brussels (BE)
(86) International application number: PCT/EP2009/052454
(87) International publication number: WO 2009/109547

(56) References cited:
- EP-A- 1 731 149
- WO-A-01/62726
- WO-A-2005/121082
- THEUER HAGEN ET AL: "Stabilitätsuntersuchungen von Piracetam-Infusionslösungen" PHARMAZEUTISCHE ZEITUNG, FRANKFURT, DE, vol. 132, no. 17, 1 January 1987 (1987-01-01), pages 1024-1029, XP002171959 ISSN: 0031-7136

## Description

The present invention concerns stable liquid formulations of brivaracetam, a process of the preparation thereof and therapeutic uses thereof.

International patent application having publication number WO 01/62726 discloses 2-oxo-1-pyrrolidine derivatives and methods for their preparation. It particularly discloses compound (2S)-2-[(4R)-2-oxo-4-propyl-pyrrolidin-1-yl] butanamide known under the international non propriety name of brivaracetam.

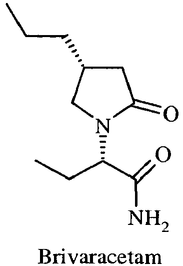

EP1731149 discloses the use of brivaracetam for the prevention or treatment of progressive myoclonic epilepsy. A number of pharmaceutical compositions is suggested, including solutions.

International patent application having publication number WO 2005/121082 describes a process of preparation of 2-oxo-1-pyrrolidine derivatives and particularly discloses a process of preparation of (2S)-2-[(4S)-4-(2,2-difluorovinyl)-2-oxo-pyrrolidin-1-yl]butanamide known under the international non propriety name of seletracetam.

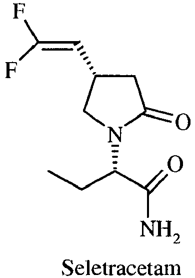

2-oxo-1-pyrrolidine derivatives are therefore particularly useful in the pharmaceutical industry.

Brivaracetam is effective in the treatment of epilepsy. Brivaracetam is also effective in the treatment of patients with refractory partial onset seizures, with or without secondary generalization. In the therapeutic confirmatory Phase III studies the efficacy and safety of brivaracetam are tested at doses of 5 to 100 mg per day in the adjunctive treatment of adult patients (16-65 years). Brivaracetam has also an indication in the treatment of Progressive Myoclonic Epilepsy and of Symptomatic Myoclonus.

Seletracetam is effective in the treatment of epilepsy.

Until now, brivaracetam and seletracetam have been formulated in solid compositions (film coated tablet, granules).

However, an oral solution would be particularly desirable for administration in children and also in some adult patients. An injectable solution could be advantageously used in case of epilepsy crisis.

Moreover, administration of an oral dosage form is the preferred route of administration for many pharmaceuticals because it provides for easy, low-cost administration. However some patients such as children or elderly people can have problems when requested to swallow a solid formulation such as a tablet or a capsule. Hence the development of a liquid oral formulation is therefore desirable since it offers improved patient compliance.

However, stability storage tests have shown that aqueous solutions of 2-oxo-1-pyrrolidine derivatives were partially unstable. During these tests, degradation products in solution are formed by basic or acid hydrolysis, in fact an epimerisation and/or amide hydrolysis occurred, but also oxidation, with detection of hydroxyamide and hydroxyacid impurities.

It has now surprisingly been found that these degradation products are not formed at pH values between 4.5 and 6.5. In fact kinetics of degradation is the slowest in normal conditions (room temperature) when the drug solution has a pH value of between 4.5 and 6.5.

The invention relates to a stable solution of a pharmaceutical compound being (2S)-2-{(4R)-2-oxo-4-propyl-pyrrolidin-1-yl}} butanamide, characterized in that it has a pH value of between 4.5 and 6.5.

Preferably, the solution of the invention has a pH values between 5.0 and 6.0. The best results are obtained with a pH value of about 5.5.

By "stable" we mean optimum of stability in normal condition of storage (room temperature).

In particular, the invention relates to an injectable solution or an oral solution. When it is an injectable solution, the solution has preferably a pH value of 5.5 ± 0.2. When it is an oral solution, the solution has preferably a pH value of 5.5 ± 0.2.

The amount by weight of the pharmaceutical compound in an injectable solution is generally in the range of 0.01 mg per ml to 200 mg per ml; and preferably of 0.1 mg to 50 mg per ml; and more preferably of 1 mg to 30 mg per ml.

The amount by weight of the pharmaceutical compound in an oral solution is generally in the range of 0.01 mg per ml to 100 mg per ml; preferably of 0.1 mg to 50 mg per ml; and more preferably of 1 mg to 20 mg per ml.

Usually, the solution is aqueous or alcoholic. In a preferred embodiment of the invention, the solution is an aqueous solution: water is used as solvent, preferably purified water for an oral aqueous solution and water for injection and pyrogen-free for the injectable form.

The solution can be administered directly intravenously, intramuscular or parenterally, or designed as infusion solutions or concentrates as supplements to infusions.

Substances for adjusting the pH value are physiological buffers. The pH of the compositions is maintained by a buffer system. Buffer systems comprise mixtures of appropriate amounts of an acid such as phosphoric, succinic, tartaric, lactic, or citric acid, and a base, in particular sodium hydroxide or disodium hydrogen phosphate. Ideally, the buffer has sufficient capacity to remain in the intended pH range upon dilution with a neutral, a slightly acidic or a slightly basic beverage.

Examples of buffers are acetic acid, phosphate and citric acid. The best results are obtained with acetic acid and citric acid.

Pharmaceutically acceptable excipients may be added to the solution, such as preservatives and formulation agents. Preservatives are included in preparations to kill or inhibit the growth of micro-organisms inadvertently introduced during manufacture or use and are therefore essential ingredients. The choice of a suitable preservative for a preparation depends on pH, compatibility with other ingredients, the route of administration, dose and frequency of administration of the preparation, partition coefficients with ingredients and containers or closures, degree and type of contamination, concentration required, and rate of antimicrobial effect

The present invention concerns also a process for the production of a stable solution wherein a solution of the pharmaceutical compound is adjusted to a pH value of between 4.5 and 6.5.

According to the invention, the solution also may contain sodium chloride or sodium acetate for the injectable form and sweeteners, flavours, palatability agents for the oral forms. Furthermore, the general perception of sweetness and taste were improved

The pharmaceutically acceptable sweeteners comprise preferably at least one intense sweetener such as saccharin, sodium or calcium saccharin, aspartame, acesulfame potassium, sodium cyclamate, alitame, a dihydrochalcone sweetener, monellin, stevioside or sucralose (4,1',6'-trichloro-4,1',6'-trideoxygalactosucrose), preferably saccharin, sodium or calcium saccharin, and optionally a bulk sweetener such as sorbitol, mannitol, fructose, sucrose, maltose, isomalt, glucose, hydrogenated glucose syrup, xylitol, caramel or honey.

The intense sweetener is conveniently employed in low concentrations. For example, in the case of sodium saccharin, the concentration may range from 0.01% to 0.1% (w/v) based on the total volume of the final formulation, and preferably is about 0.05% (w/v).

The bulk sweetener, such as sorbitol, can effectively be used in larger quantities ranging from about 10% to about 35% (w/v, weight/volume), preferably from about 15% to 30% (w/v), more preferably about 25 % (w/v).

When sorbitol is used as a bulk sweetener it is preferably used as an aqueous solution containing 70% (w/w) of sorbitol.

The pharmaceutically acceptable flavours which can mask the bitter tasting ingredients in the low-dosage formulations are preferably fruit flavours such as cherry, raspberry, black currant, strawberry flavour, caramel chocolate flavour, mint cool flavour, fantasy flavour and the like pharmaceutically acceptable strong flavours. Each flavour may be present in the final composition in a concentration ranging from 0.05% to 1% (w/v).

Combinations of said strong flavours are advantageously used. Preferably a flavour is used that does not undergo any change or loss of taste and colour under the acidic conditions of the formulation.

Preferably; the injectable solution contains sodium chloride.

To prepare the solutions, 80 % of the requisite amount of water is prepared and pharmaceutical compound and the other excipients are dissolved by stirring. When dissolution is complete, the pH is verified and adjusted if necessary to the desired pH, preferably about 5.5 (+/- 0.5). This solution is made up to the final volume with water.

For the injectable form, the solution obtained in this manner is sterilized by filtration through conventional pathogen-proof filters and then dispensed into appropriate containers for injectable preparations (ampoules or vials) and post-sterilized. The water used in the process of preparation is sterile and is pyrogen-free.

The oral solutions are filtered on appropriate filters and dispensed in appropriate containers for oral administration.

The present invention also concerns a use of a stable solution for the manufacture of a medicament for a therapeutic application.

The present invention also concerns a use of a stable solution for the treatment of disease.

The present invention concerns also a method for treating a patient comprising administering to such a patient a therapeutically effective amount of a stable solution.

The present invention concerns also a pharmaceutical composition comprising a stable solution with a pH value of between 4.5 and 6.5.

The present invention concerns also a liquid pharmaceutical preparation comprising a stable brivaracetam solution with a pH value of between 4.5 and 6.5, this preparation containing less than 0.2 % (by weight) of impurities (impurities including degradation products).

The oral solution of the invention is particularly useful for administration in children or in adult patients for whom administration with tablets is not feasible.

Another advantage of the invention resides in the fact that the injectable solution permits rapid interventions in cases of emergency or crisis, or for those patients for whom administration with any formulation through oral intake is not feasible. These characteristics of brivaracetam make it ideal for administration in liquid forms, contrasted by most other drugs, with the same indications, which are very poorly soluble in water.

The following examples illustrate the invention without however limiting its scope.

Example 1. Brivaracetam solutions - 20 mg/ml - 1 ml of solution in 1.5 ml sealed glass vials.

Solutions at different pH are prepared (natural pH (not buffered) and pH 4.5, 5.0, 5.5, 6.0. The pH is controlled by means of an adequate buffer in order to obtain the wished pH (in the example by 50 mM citrate).

The solution is dispensed in 1.5 ml sealed glass vials.

A stability test is performed at 25°C, 40 °C, 60 °C and 80 °C.

The pH of the various solutions is measured at the beginning of the test and after 2 weeks, 4 weeks, and 10 weeks. The amounts of degradation products in the brivaracetam solutions are dosed in the various solutions.

The results are summarized in tables 1, 2 and 3 as follows.

**Table 1: sum of all degradation products detected (% relative areas) after 2 weeks of stability at 25 °C, 40 °C, 60 °C and 80 °C**

| Initial pH | at 25°C | at 40 °C | at 60 °C | at 80 °C |
|---|---|---|---|---|
| 4.6 | 0.0 | 0.0 | 0.3 | 2.0 |
| 5.1 | 0.0 | 0.0 | 0.1 | 0.7 |
| 5.6 | 0.0 | 0.0 | 0.0 | 0.5 |
| 6.1 | 0.0 | 0.0 | 0.0 | 1.4 |

**Table 2: sum of all degradation products detected (% relative areas) after 4 weeks of stability at 25 °C, 40 °C, 60 °C and 80 °C**

| Initial pH | at 25°C | at 40 °C | at 60 °C | at 80 °C |
|---|---|---|---|---|
| 4.7 | 0.0 | 0.1 | 0.8 | 4.3 |
| 5.2 | 0.0 | 0.0 | 0.2 | 1.4 |
| 5.7 | 0.0 | 0.0 | 0.1 | 1.1 |
| 6.2 | 0.0 | 0.0 | 0.1 | 2.9 |

**Table 3: sum of all degradation products detected (% relative areas) after 10 weeks of stability at 25 °C, 40 °C, 60 °C and 80 °C**

| Initial pH | at 25°C | at 40 °C | at 60 °C | at 80 °C |
|---|---|---|---|---|
| 4.7 | 0.0 | 0.1 | 1.7 | 10.7 |
| 5.2 | 0.0 | 0.0 | 0.5 | 3.7 |
| 5.7 | 0.0 | 0.0 | 0.1 | 2.7 |
| 6.1 | 0.0 | 0.0 | 0.2 | 7.6 |

These results show that brivaracetam solution is stable in the range of pH of 4.5 and 6.5. These results demonstrate clearly that the degradation rate is the lowest for a solution having a pH range of 5.0 and 6.0.

### Example 2 (not according to the invention) Seletracetam solutions - 10 mg/ml - 1 ml of solution in 1.5 ml sealed glass vials.

Solutions at different pH are prepared (natural pH (not buffered) and pH 4.5, 5.0, 5.5, 6.0. The pH is controlled by means of an adequate buffer in order to obtain the wished pH (in the example: 50mM acetate).

The solution is dispensed in 1.5 ml sealed glass vials.

A stability test is performed at 25°C, 40 °C, 60 °C and 80 °C.

The pH of the various solutions is measured at the beginning of the test and after 2 weeks, 4 weeks, and 10 weeks. The amounts of degradation products in the seletracetam solutions are dosed in the various solutions.

The results are summarized in the following tables.

**Table 4: sum of all degradation products detected (% relative areas) after 2 weeks of stability at 25 °C, 40 °C, 60 °C and 80 °C**

| Initial pH | at 25°C | at 40 °C | at 60 °C | at 80 °C |
|---|---|---|---|---|
| 4.6 | 0.0 | 0.0 | 0.0 | 1.0 |
| 5.1 | 0.0 | 0.0 | 0.0 | 0.3 |
| 5.7 | 0.0 | 0.0 | 0.0 | 0.5 |
| 6.1 | 0.0 | 0.0 | 0.0 | 1.4 |

**Table 2: sum of all degradation products detected (% relative areas) after 4 weeks of stability at 25 °C, 40 °C, 60 °C and 80 °C**

| Initial pH | at 25°C | at 40 °C | at 60 °C | at 80 °C |
|---|---|---|---|---|
| 4.7 | 0.0 | 0.0 | 0.1 | 1.7 |
| 5.2 | 0.0 | 0.0 | 0.1 | 0.9 |
| 5.7 | 0.0 | 0.0 | 0.0 | 1.0 |
| 6.2 | 0.0 | 0.0 | 0.0 | 2.7 |

**Table 3: sum of all degradation products detected (% relative areas) after 10 weeks of stability at 25 °C, 40 °C, 60 °C and 80 °C**

| Initial pH | at 25°C | at 40 °C | at 60 °C | at 80 °C |
|---|---|---|---|---|
| 4.7 | 0.0 | 0.1 | 1.0 | 5.8 |
| 5.2 | 0.0 | 0.0 | 0.3 | 3.4 |
| 5.7 | 0.0 | 0.0 | 0.4 | 3.0 |
| 6.1 | 0.0 | 0.0 | 0.4 | 7.8 |

These results show that seletracetam solution is stable in the range of pH of 4.5 and 6.5. These results demonstrate clearly that the degradation rate is the lowest for a solution having a pH range of 5.0 and 6.0.

### Example 3: Injectable solution of brivaracetam - 50 mg/ml - Vial

The composition of the solution is as follows:

| | | |
|---|---|---|
| Brivaracetam | | 50 mg |
| Sodium acetate | | 13.5 mg |
| Glacial acetic acid | | q.s. for pH = 5.5 |
| Sodium chloride | | 45 mg |
| Water for injection | q.s. for | 5 ml |

| | | |
|---|---|---|
| Brivaracetam, sodium chloride and sodium acetate are dissolved in 80 % of the quantity of water for injection. The pH is adjusted to 5.5 by means of a 0.1 N acetic acid solution. The required volume is completed with water for injection. The solution is filtered on a 0.22 µm filter preceded of a pre-filter. Glass vials 6ml are filled. Sealed ampoules or closed vials are sterilized by steam sterilization (autoclave 20 minutes, 121°C). The injectable solution of brivaracetam is very easy to prepare and contains no excessive excipient. | | |

### Example 4. Oral solution 1 mg/ml - Brivaracetam

The composition of the solution is as follows:

| | | |
|---|---|---|
| Compounds | | mg |
| Brivaracetam | | 5.00 |
| Water purified | | 3000.00 |
| Methylparaben | | 5.00 |
| Citric acid | | 4.475 |
| Sodium citrate dihydrate | | 14.70 |
| Sodium carboxymethylcellulose | | 25.00 |
| Sucralose | | 20.00 |
| Sorbitol solution | | 1199.00 |
| Glycerol | | 760.00 |
| flavor | | 28.00 |
| Purified water | ad | 5.00 ml |

| | | |
|---|---|---|
| In a stainless tank, 90 % of the glycerol is transferred and methylparaben are added. Dissolution is obtained by heating while stirring speed. In another tank, purified water is transferred and sodium citrate, citric acid are dissolved. Brivaracetam is added while stirring until complete dissolution is reached. Both solutions are mixed. Water is added to the final volume and the preparation is homogenized. The pH is controlled (pH = 5.6 ± 0,3) with a pHmeter. The preparation is filtered on a 40 µm cartridge filter. | | |

### Example 5 (not according to the invention) Intravenous solution of seletracetam - 100 mg/ml - vial

The composition of the solution is as follows.

| | |
|---|---|
| Seletracetam | 100 mg |
| Sodium acetate | 2.7 mg |
| Sodium Chloride | 9 mg |
| Glacial acetic acid | q.s. for pH = 5.5 |
| Water for injection | q.s. for 1 ml |

| | |
|---|---|
| Seletracetam, sodium chloride and sodium acetate are dissolved in 80% of the quantity of water for injection. The pH is adjusted to 5.5 by means of a 0.1 N acetic acid for injection. The required volume is completed with water for injection. Sealed ampoules or closed vials are sterilized by steam sterilization (Autoclave, 30 minutes, 121°C). | |

### Example 6 - Brivaracetam Oral Solution 10 mg/ml

The composition of the solution is as follows.

| | |
|---|---|
| Components | mg/ml |
| Methylparaben | 1 |
| Citric Acid | 0.895 |
| Sodium Citrate Dihydrate | 2.94 |
| Sodium Carboxymethylcellulose (Blanose®) | 5 |
| Brivaracetam | 10 |
| Sucralose | 40 |
| Sorbitol Solution | 239.8 |
| Glycerin, | 152 |
| Flavor, Artificial (Raspberry) | 5.6 |
| Water Purified | Qs |

| | |
|---|---|
| The oral solution is prepared as described in example 4. The pH is controlled (pH = 5.4 ± 0.2) with a pHmeter. The oral solution is stable. Moreover, it is an organoleptically acceptable oral aqueous solution. | |

### Example 7 - Brivaracetam Oral Solution 1 mg/ml

The composition of the solution is as follows.

| Components | mg/ml |
|---|---|
| Methylparaben, | 1 |
| Citric Acid | 0.895 |
| Sodium Citrate Dihydrate, | 2.94 |
| Sodium Carboxymethylcellulose (Blanose®) | 5 |
| Brivaracetam | 1 |
| Sucralose | 4 |
| Sorbitol Solution | 239.8 |
| Glycerin, | 152 |
| Flavor, Artificial (Raspberry) | 5.6 |
| Water Purified | Qs |

| | |
|---|---|
| The oral solution is prepared as described in example 4. The pH is controlled (pH = 5.5 ± 0.2) with a pHmeter. The oral solution is stable. | |

### Example 8 : LBS Binding Assay

[LBS stands for Levetiracetam Binding Site cf. M. Noyer et al., Eur. J. Pharmacol., 286 (1995) 137-146.]

The inhibition constant (Kᵢ) of a compound is determined in competitive binding experiments by measuring the binding of a single concentration of a radioactive ligand at equilibrium with various concentrations of the unlabeled test substance. The concentration of the test substance inhibiting 50 % of the specific binding of the radioligand is called the IC₅₀. The equilibrium dissociation constant Ki is proportional to the IC₅₀ and is calculated using the equation of Cheng and Prusoff (Cheng Y. et al., Biochem. Pharmacol. 1972, 22, 3099-3108).

The concentration range usually encompasses 6 log units with variable steps (0.3 to 0.5 log). Assays are perfomed in mono- or duplicate, each Kᵢ determination is performed on two different samples of test substance.

Cerebral cortex from 200-250g male Sprague-Dawley rats are homogenised using a Potter S homogeniser (10 strokes at 1,000 rpm; Braun, Germany) in 20 mmol/l Tris-HCl (pH 7.4), 250 mmol/l sucrose (buffer A); all operations are performed at 4 °C. The homogenate is centrifuged at 30,000g for 15 min. The crude membrane pellet obtained is resuspended in 50 mmol/l Tris-HCl (pH 7.4), (buffer B) and incubated 15 min at 37 °C, centrifuged at 30,000xg for 15 min and washed twice with the same buffer. The final pellet is resuspended in buffer A at a protein concentration ranging from 15 to 25 mg/ml and stored in liquid nitrogen.

Membranes (150-200 µg of protein / assay) are incubated at 4 °C for 120 min in 0.5 ml of a 50 mmol/l Tris-HCl buffer (pH 7.4) containing 2 mmol/l MgCl₂ , 1 to 2 10⁻⁹ mol/l of [³H]-2-[4-(3-azidophenyl)-2-oxo-1-pyrrolidinyl]butanamide and increasing concentrations of the test substance. The non specific binding (NSB) is defined as the residual binding observed in the presence of a concentration of reference substance (e.g. 10⁻³ mol/l levetiracetam) that binds essentially all the receptors. Membrane-bound and free radioligands are separated by rapid filtration through glass fiber filters (equivalent to Whatman GF/C or GF/B; VEL, Belgium) pre-soaked in 0.1 % polyethyleneimine and 10⁻³ mol/l levetiracetam to reduce non specific binding. Samples and filters are rinsed by at least 6 ml of 50 mmol/l Tris-HCl (pH 7.4) buffer. The entire filtration procedure does not exceed 10 seconds per sample. The radioactivity trapped onto the filters is counted by liquid scintillation in a β-counter (Tri-Carb 1900 or TopCount 9206, Camberra Packard, Belgium, or any other equivalent counter). Data analysis is perfomed by a computerized non linear curve fitting method using a set of equations describing several binding models assuming populations of independent non-interacting receptors which obey to the law of mass.

Compounds according to the invention, and in particular brivaracetam and seletracetam, showed pKi values of 6.0 and greater.

### EXAMPLE 9 : Animal model of sound-susceptible mice

The objective of this test is to evaluate the anticonvulsant potency of a compound in sound-susceptible mice, a genetic animal model with reflex seizures. In this model of primary generalised epilepsy, seizures are evoked without electrical or chemical stimulation and the seizure types are, at least in part, similar in their clinical phenomenology to seizures occuring in man (Löscher W. & Schmidt D., Epilepsy Res. (1998), 2, 145-181; Buchhalter J.R., Epilepsia (1993), 34, S31-S41).

Male or female genetically sound-sensitive mice (14-28 g; N=10), derived from a DBA strain originally selected by Dr. Lehmann of the Laboratory of Acoustic Physiology (Paris) and bred in the UCB Pharma Sector husbandry unit since 1978, are used. The experimental design consisted of several groups, one group receiving the vehicle control and the other groups different doses of the test-compound. The compounds are administered intraperitoneally 60 minutes before the induction of audiogenic seizures. The range of the doses administered had a logarithmic progression, generally between 1.0 x 10⁻⁵ mol/kg and 1.0 x 10⁻³ mol/kg, but lower or higher doses are tested if necessary.

For testing, the animals are placed in small cages, one mouse per cage, in a sound-attenuated chamber. After a period of orientation of 30 seconds, the acoustic stimulus (90dB, 10-20 kHz) is delivered for 30 seconds via loudspeakers positioned above each cage. During this interval, the mice are observed and the presence of the 3 phases of the seizure activity namely wild running, clonic and tonic convulsions, is recorded. The proportion of mice protected against wild running, clonic and tonic convulsions, respectively, is calculated.

For active compounds, an ED50 value, i.e. the dose producing 50 % protection relative to the control group, together with 95% confidence limits, was calculated using a Probit Analysis (SAS/STAT® Software, version 6.09, PROBIT procedure) of the proportions of protected mice for each of the 3 phases of the seizure activity.

Compounds according to the invention, and in particular brivaracetam and seletracetam, showed ED50 values of 1.0E-04 or lower.

### Example 10: bioavailability and safety of an intravenous formulation of brivaracetam

Brivaracetam is an antiepileptic drug with high affinity toward the synaptic vesicle protein SV2A.

A first step of the study done in a 3-way crossover in 24 healthy subjects (12 females, 12 males) compares the single-dose bioavailability of brivaracetam 10 mg oral tablets versus brivaracetam 10 mg administered as a 15 minute intravenous infusion, and as an IV bolus. In a second step, a single-dose escalation study (25 mg, 50 mg, 100 mg and 150 mg) is performed in 4 consecutive groups of 6 subjects (3 females, 3 males) to gain information and as a fast IV bolus, to assess the pharmacokinetics of brivaracetam at these dosing regimens and to explore dose proportionality.

Brivaracetam 10 mg administered either via a 15-minute IV infusion or via an IV bolus is bioequivalent to a single dose of brivaracetam 10 mg oral tablet (90 % confidence intervals of geometric ratios of both Cmax and AUC were fully contained within the bioequivalence range of 80-125%).

Brivaracetam (from 25 mg to 150 mg) administered as IV infusion or IV bolus is safe and well tolerated. Pharmacokinetic parameters of brivaracetam after IV infusion and IV bolus were similar.Extends of exposure are proportional to the administered dose in the dose range (25-150 mg).

## Claims

1. A stable solution of a pharmaceutical compound being (2S)-2-{(4R)-2-oxo-4-propyl-pyrrolidin-1-yl}} butanamide, **characterized in that** it has a pH value of between 4.5 and 6.5.

2. The solution according to claim 1, **characterized in that** the pH values are between 5.0 and 6.0.

3. The solution according to claim 1, **characterized in that** it is an injectable solution, the amount of the pharmaceutical compound being in the range of 0.01 mg per ml to 200 mg per ml.

4. The solution according to claim 1, **characterized in that** it is an oral solution, the amount of the pharmaceutical compound being in the range of 0.01 mg per ml to 100 mg per ml.

5. The solution according to claim 1, **characterized in that** it is an injectable solution having a pH value of 5.5 ± 0.2.

6. The solution according to claim 1, **characterized in that** it is an oral solution having a pH value of 5.5 ± 0.2.

## Patentansprüche

1. Stabile Lösung einer pharmazeutischen Verbindung, welche (2S)-2-{(4R)-2-Oxo-4-propyl-pyrrolidin-1-yl}}butanamid ist, **dadurch gekennzeichnet, dass** sie einen pH-Wert zwischen 4,5 und 6,5 hat.

2. Lösung nach Anspruch 1, **dadurch gekennzeichnet, dass** die pH-Werte zwischen 5,0 und 6,0 liegen.

3. Lösung nach Anspruch 1, **dadurch gekennzeichnet, dass** es sich um eine injizierbare Lösung handelt, wobei die Menge der pharmazeutischen Verbindung im Bereich von 0,01 mg pro ml bis 200 mg pro ml liegt.

4. Lösung nach Anspruch 1, **dadurch gekennzeichnet, dass** es sich um eine orale Lösung handelt, wobei die Menge der pharmazeutischen Verbindung im Bereich von 0,01 mg pro ml bis 100 mg pro ml liegt.

5. Lösung nach Anspruch 1, **dadurch gekennzeichnet, dass** es sich um eine injizierbare Lösung mit einem pH-Wert von 5,5 ± 0,2 handelt.

6. Lösung nach Anspruch 1, **dadurch gekennzeichnet, dass** es sich um eine orale Lösung mit einem pH-Wert von 5,5 ± 0,2 handelt.

## Revendications

1. Solution stable d'un composé pharmaceutiques qui est le (2S)-2-[(4R)-2-oxo-4-propyl-pyrrolidine-1-yl)] butanamide, **caractérisée en ce qu'**elle a un pH compris entre 4,5 et 6,5.

2. Solution suivant la revendication 1, **caractérisée en ce que** les pH sont compris entre 5,0 et 6,0.

3. Solution suivant la revendication 1, **caractérisée en ce que** c'est une solution injectable, la quantité du composé pharmaceutique étant dans la plage allant de 0,01 mg par ml à 200 mg par ml.

4. Solution suivant la revendication 1, **caractérisée en ce que** c'est une solution orale, la quantité du composé pharmaceutique étant dans la plage allant de 0,01 mg par ml à 100 mg par ml.

5. Solution suivant la revendication 1, **caractérisée en ce que** c'est une solution injectable ayant un pH de 5,5 ± 0,2.

6. Solution suivant la revendication 1, **caractérisée en ce que** c'est une solution orale ayant un pH de 5,5 ± 0,2.
